# EUROPEAN PATENT SPECIFICATION

(11) **EP 4 380 506 B1**
(45) Date of publication and mention of the grant of the patent: **27.08.2025**
(21) Application number: 22754555.5
(22) Date of filing: 28.07.2022
(51) Int. Cl.: A61F 2/54, A61F 2/58, A61F 2/70, A61F 2/72, A61F 2/80, A61F 2/50, A61F 2/68

(54) **EVOLVED PROSTHETIC SOCKET FOR UPPER LIMB PROSTHESES**
EVOLVED-PROTHETIKSCHAFT FÜR PROTHESEN DER OBEREN GLIEDMASSEN
EMBOÎTURE PROTHÉTIQUE ÉVOLUÉE POUR PROTHÈSES DE MEMBRES SUPÉRIEURS

(30) Priority: 04.08.2021 IT 202100021038
(43) Date of publication of application: 12.06.2024
(73) Proprietor: BIONIT LABS SRL, 73010 Soleto (LE) (IT)
(72) Inventor: BIANCO, Fabio, 73013 Galatina (LE) (IT); GAETANI, Federico, 73049 Ruffano (LE) (IT)
(74) Representative: Conversano, Gabriele
(86) International application number: PCT/IB2022/057000
(87) International publication number: WO 2023/012613

(56) References cited:
- US-A1- 2017 203 432
- US-A1- 2019 091 040
- GAETANI FEDERICO ET AL: "Hardware design and software development of a motion control and driving system for transradial prosthesis based on a wireless myoelectric armband", 1 May 2019, IET SCIENCE, MEASUREMENT AND TECHNOLOGY, THE INSTITUTION OF ENGINEERING AND TECHNOLOGY, MICHAEL FARADAY HOUSE, SIX HILLS WAY, STEVENAGE, HERTS. SG1 2AY, UK, PAGE(S) 354 - 362, ISSN: 1751-8822, XP006087231

## Description

### Technical field of the Invention

The present invention finds its application, in general, in the context of the manufacture and supply of prostheses for upper limbs equipped with an artificial hand. In particular, the solution concerns some innovative features of the prosthetic socket: that is, the element that acts as a connection between the patient's stump and the actual artificial hand.

### State of the Art

The hand is a part of the human body that is used to perform a very wide variety of operations. Also for this reason, the upper limbs are affected by numerous accidents, some of which, as a consequence, involve the amputation of the limb. The hand is so important for the execution of many operations, to the point that, when a person lacks a hand or even part of the arm, an artificial prosthesis is normally used (for further information, see the extensive literature on the subject; see also: 2004, Weir, Standard Handbook of Biomedical Engineering & Design, "DESIGN OF ARTIFICIAL ARMS AND HANDS FOR PROSTHETIC APPLICATIONS", https://www.researchgate.net/publication/ 303819177 Standard Handbook of Biomedical Engineering Design).

This practice of applying artificial prostheses to replace at least part of the functionality of the hand is a practice that has its roots in the past. Evidently the prostheses of the past were very rudimentary and not comparable in the least with the functionality of a natural hand, nevertheless they were still applied, and patients learned to use such prostheses. Sometimes, a simple hook was applied instead of the hand to perform some simple operations such as pulling or pushing an object.

Today, extremely sophisticated artificial hands can be made, which offer relatively complex functionalities, with the aim of allowing patients to perform as many operations as possible, **similar** to those they could perform with their natural hands.

Hence, for the patient; the problem arises of having the practical control of an artificial hand, so sophisticated that it requires articulated and complex commands.

It is also observed that the control of the hand is particularly complex as this is not just characterized by operational functions, but also by sensory functions.

Therefore, it can be stated that, in the ideal case, the control of a hand must be "bidirectional", in the sense that the patient's brain must be able to give commands to the hand to perform operational tasks (therefore the brain must transmit commands towards the hand), but also, the brain must receive sensory feedback from the hand, that is, it must receive information from the hand.

This theme, namely the importance of "proprioception", is very broad, it has been studied for a long time and is treated by numerous studies, still at the research level, with the aim of providing the prosthetic limbs with some proprioceptive features typical of natural limbs (see 2003, IEEE International Conference on Robotics and Automation, Carrozza et al., "EXPERIMENTAL ANALYSIS OF AN INNOVATIVE PROSTHETIC HAND WITH PROPRIOCEPTIVE SENSORS", https://ieeexplore.ieee.org/abstract/document/1241925).

For the purposes of the presentation of the present invention, it is only necessary to highlight the fact that progress is also desirable in reference to the improvement of "proprioception", and therefore it may be important to have prosthetic systems conceived (in their architecture) in such a way that it can be as easy as possible to integrate new measures aimed at improving the management of sensory feedback that can be provided to the patient.

Just these simple observations bring into focus a first problem of the prosthesis of a hand: that is the complexity of the interface between the artificial hand and the natural body of the patient, and in particular the stump to which the hand (and at least a part forearm) must be applied.

For the purposes of the present invention, in fact, it is assumed that the artificial hand is a known device equipped with numerous functional and sensory functions, and that the main limit is given by the possibility of control by the patient.

As regards the connection between the artificial hand and the patient's stump, it is also important to consider the aesthetic aspect (for more information on the subject, see also: Prosthetics and Orthotics International, Ritchie et al., "PROSTHETICS AND ORTHOTICS INTERNATIONAL, PERCEPTIONS OF COSMESIS AND FUNCTION IN ADULTS WITH UPPER LIMB PROSTHESES: A SYSTEMATIC LITERATURE REVIEW", https://journals.sagepub.com/doi/pdf 10.1177/0309364611420326).

Although aesthetic issues are in the background to functional ones, it is right to offer patients who have lost a hand, or patients who have been without it since birth due to malformations, a solution that is sufficiently treated also from an aesthetic point of view.

Therefore, while the strictly intended artificial hand can be industrially produced in a few sizes, substantially independently of the patient's conformation, the connecting element must necessarily be made to measure in a personalized way.

In fact, it is necessary to adapt to the stump, so that both the natural arm and the prosthetic arm have approximately the same size. In addition, the whole prosthesis as a whole must be comfortable and easy to use, in order to facilitate use by the patient, so as to avoid his movements being too clumsy and excessively highlighting the disability.

Ultimately, the known art proposes prostheses for upper limbs generally composed of two integrated systems.
✔ A first system consists of a mechanical artificial hand, which we will not particularly focus on in the context of this description, assuming that this system is available and that it can support both operational functions and sensory functions (understood in the sense of the production of feedback returned to the patient).
✔ A second system consists of the connecting element between the aforementioned artificial hand and the patient's body, which in this specific case is the stump of a forearm. This second system is called, in the jargon used in the sector, "prosthetic socket" and it is an element that must be made to measure by an orthopedic technician.

The present invention, as will be clarified further below, indicates some innovative features concerning the system consisting of the prosthetic socket, with the aim of preserving current practice. In fact, the solution proposed in the present invention also provides for maintaining the modular distinction of the two systems indicated above; distinction that allows you to implement an efficient process of production and supply of even highly customized prostheses.

It should be noted that the invention proposes some teachings that can be applied in various types of prostheses, and in particular also in upper limb prostheses in which the patient is missing almost all of his upper limb, and the prosthesis must also include the upper limb. elbow joint (transhumeral amputations) or the whole upper limb, and the prosthesis must also include the shoulder joint (shoulder disarticulations).

In this case, the mechanical part is necessarily more complex as it must reproduce not just a hand and the wrist, but also the elbow and possibly the shoulder: therefore, the prosthetic socket must be applied to a stump near the shoulder or the torso of the patient. Even in the latter cases, however, some general characteristics of the prosthetic socket, which will be described below, as the subject matter of the invention, will be substantially applicable, albeit with a greater degree of complexity.

For the sake of convenience, a description will be given below, referring to the simplest case in which the prosthesis consists of an artificial mechanical hand, possibly including the wrist joints (all or just some), and the prosthetic socket, which completes the forearm of the patient and performs the aforementioned connecting functions.

It should be noted that, in the following, the expressions artificial "mechanical hand" or "mechanical limb" will be used, to mean that part of the prosthesis that includes mechanisms that reproduce the mechanical and mobility functions of the artificial limb, assuming that, in reality, these are electromechanical and electronic objects, as they include electric actuators, sensors, and electronic components in general.

Obviously, we do not intend some prostheses, now obsolete but still used, purely mechanical in which the motor functions were carried out through mechanisms implemented with belts, tie rods, lever systems, etc. From now on, therefore, the term "mechanical limb", or "mechanical hand", means a prosthesis with mechanical functionality, but of modern design, therefore electrically powered and controlled through appropriate control electronics.

Unlike state-of-the-art "mechanical limbs", which are extremely sophisticated systems, prosthetic sockets according to the known art are generally very simple systems. In fact, having to be substantially artisanal elements, as they are tailor-made, the simpler they are, the easier and more reliable their manufacture is.

The orthopedic technician must concentrate mainly on morphological aspects while, from the point of view of technical components, the orthopedic technician who makes the socket must essentially limit himself to managing the power supply, and two or more sensors (depends on the residual muscle function of the abutment) able to detect the contraction of some muscles accessible at the end of the stump.

The voluntary contraction of some residual muscles in correspondence with the stump is in fact the main action that can be performed by the patient to exercise operational control of the prosthesis. The patient must therefore learn a kind of new language to control his artificial limb, and by contracting the muscles of his stump with particular sequences, he can learn to perform some simple operations with his artificial hand.

Obviously, these contraction sensors must be positioned in the part of the prosthetic socket that physically touches the abutment, therefore in the farthest point from the artificial hand which is attached to the opposite end of the socket itself, and therefore the exit of these sensors must be carried with short cables to the attachment point of the artificial hand, which will obviously be equipped with physical contacts to receive, as input, the commands conveyed through the aforementioned cables.

The sensors that detect the contractions of the abutment muscles are extremely small, while the wiring can be passed inside the prosthetic socket without particular difficulties.

The whole prosthetic system (the socket with its sensors, and the mechanical hand) obviously requires a power supply, which is supplied with suitable batteries. In the most frequent forms of implementation, the batteries are positioned inside the prosthetic socket, where there is room, and also provide power to the hand. Other wiring therefore serves to ensure power supply where it is needed, but, also in this case, it is a space-saving wiring that can easily be contained in the prosthetic socket.

A further feature, whose management involves the prosthetic socket, concerns sensory functionality.

It must be said that the main feedback that the patient receives from his limb is obtained from visual observation. In fact, patients learn to always look at what they are doing with their artificial limb, not being able to count on a haptic function as evolved as that offered by the natural hand.

However, some prosthetic systems provide physical feedback reactions, in various forms, to some operative actions, in order to provide some simple sensory function.

Some solutions, already proposed by the known art, are in fact suitable for implementing some sensory feedbacks, and have been used (although not yet widely) in some prostheses. For example, through the production of a vibration, or the generation of a slight electric shock, or by resorting to devices that exploit temperature differences, it is theoretically possible to transfer to the patient a quantitative, or only qualitative, feedback with respect to what he is doing with his artificial hand: what pressures he is exerting, with what force he is making grips, and so on.

For example, if the patient could perceive a vibration on his abutment, modulated according to the force with which he is exercising a grip, the patient himself (assuming he has appropriate tools for the operational control of his prosthesis) could adjust the proper handling of particularly fragile objects.

The vibratory reaction is in fact quite effective and can be easily interpreted by the patient. In cases where it is implemented (even if, as mentioned, this does not happen frequently), the vibration propagates through the prosthetic socket, reaching the patient's stump, at a point where he has sufficient sensitivity to perceive this vibration.

Although this form of sensory feedback is quite interesting, it is important to note that the prosthetic socket is not a very rigid element, and therefore the vibration, if generated near the hand, that is at the point where the sensory information is acquired, is a vibration which tends to diminish. It follows that the vibration transferred to the abutment cannot be adjusted with good sensitivity, and typically, the sensory signal is very approximate.

The general characteristics of the known technique, as summarized above, are only partially consolidated, so much so that both academic research and industry in the prosthetic sector are always looking for innovations that make a prosthesis more and more comfortable for a patient who needs it.

Every technical improvement, in fact, translates into an improvement in the daily life of a person with a disability, or in the possibility of offering certain solutions, which today are accessible only to a limited number of patients, to a greater number of people who need them. Innovation in the prosthetic sector, although mainly concentrated in the mechanical and electronic part of the artificial limbs, must also fully concern prosthetic sockets.

In the context of the present invention, it is therefore not intended to indicate new operational functionalities of a mechanical limb, or new physical modalities with which to return sensory feedback to a patient. Rather, it deals with the problem of managing the potential that is already offered today by the known art, as well as supporting the exploitation of new potentials, which may be developed in the near future, by resorting to the application of increasingly advanced sensor technologies, and by adapting technologies and precision mechanics materials to the prosthetic sector in general.

In order to briefly and further represent the state of the art on the availability of solutions aimed at improving the sensory feedback and control techniques of an artificial hand, and solutions aimed at optimizing the manufacture of prosthetic sockets, we cite, by way of example, some patents and patent applications (both for invention and utility model). This is a short review that has the sole purpose of testifying the evolutionary lines associated with the prostheses proposal, especially with reference to the efficient development of the prosthetic sockets; that is, the element of the prosthesis system that must be kept as simple as possible, since it must be made to measure, and is the element in direct contact with the patient's body.

JP 2010115348 (A) ["Method of manufacturing Prosthetic limb socket" - Horie Kota [JP] - May 27, 2010] describes a method for the fast manufacturing of prosthetic sockets in which the socket has no particular functional requirements, being essentially a tailor-made connecting object.

CN 102670336 (A) ["Splint tipe thigh and upper arm prosthetic socket" - Zhonghua Du [CN] - 18 September 2012] describes a prosthetic socket characterized by the fact that it can be made very quickly to measure using components that can be mass-produced and easily adapted to individual cases.

CN 207575300 (U) ["Splint tipe thigh and upper arm prosthetic socket" - Zhonghua Du [CN] - September 18, 2012] describes a classic prosthetic limb, in which the space inside the socket is used to house gears, wiring and battery; it is a utility model patent application.

WO 2018236208 (A1) ["A prosthetic limb integrated with a sensory system" - Hamzaid Nur Azah [MY] et al. - December 27, 2018] proposes a prosthetic limb, in which some sensory and operational functions for the control of movement are implemented in the area usually occupied by the prosthetic socket, but in this case the solution renounces the manufacturing simplicity of the customized parts.

US 2020306057 (A1) ["Modular prosthetic arm system" - Armenta Meza Adrian [MX] et al. - 1 October 2020] proposes a prosthetic limb with the characteristic of being modular, and which offers the advantage of reducing repair times by quickly replacing any damaged module. The abutment gripping module can be adapted to multiple abutments thanks to its pneumatic gripping system, which significantly reduces the need for customization of each prosthesis, reducing the manufacturing time and production costs of each prosthesis.

US 2021/0085491 (A1) ["System and Method for an advanced prosthetic hand" - Akhtar Aadeel [US] et al. - March 25, 2021], proposes an example of a prosthetic system for an upper limb, in which the wealth of sensory feedback is particularly taken care of, and in which particular materials are used that lend themselves to being printed with "3d printing" technologies. These materials allow to achieve various advantages: in fact, they allow the creation of custom-made prosthetic systems, they offer particularly robust and moisture-resistant performance, so as to facilitate the creation of bionic hands in which many sensors, many actuators, and the related control electronics, may be integrated.

However, the method of making these prostheses completely changes the established methodologies in the supply of prosthetic systems. The part of the prosthetic socket is in all respects an extension of the hand: it is a solution in which the various components of the system as a whole are not easily decoupled from the functional point of view, and the teachings contained in the mentioned patent application are applicable just to prostheses that include a hand with all the specific characteristics indicated in the application itself (as well as in other applications in the name of "Psyonic" of which this claims the anteriority).

Generally speaking, it can be said that the known art has not yet found an optimal balance to propose prosthetic systems for limbs in general, and for upper limbs in particular: a balance where the tailor-made parts are kept as simple as possible, while the operational and sensory functionalities of the prosthetic limb, even when they are enriched with new performances, can be concentrated in elements potentially standardizable, and therefore achievable with industrial production logic.

In fact, in the search for this balance of decoupling between standard (complex) parts and custom-made (simple) parts, in some cases, the richness of functionality has been renounced by proposing prostheses with a reduced level of controllability or with reduced sensory feedback functions; alternatively they have been proposed systems that provide for the creation of extremely complex, and therefore expensive, limb prostheses, in which also the connecting parts with the stump (i.e., the inevitably customized parts) are characterized by significant constructive complexity

It can therefore be said that there is still a need that is not yet fully satisfied by the available systems (or that can be improved in several respects). That is, the need to have modular prostheses for limbs in which standard elements, having advanced and complex functionalities, can be integrated with generality, in almost all prosthetic systems, with a substantially negligible impact on the tailor-made parts confined in the prosthetic sockets.

Here below are cited some other known art documents, which are considered somehow relevant for the purposes of the invention described in the following part of this description:
- GAETANI F. et al. - "Hardware design and software development of a motion control and driving system for transradial prosthesis based on a wireless myoelectric armband" (IET SCIENCE - Measurement and technology, Michael Faraday House, Six Hills Way, Stevanage, Herts. SG1 2AY, UK, pages 354-362, XP006087231 - ISSN: 1751-8822) 1 May 2019
- US 2017/203432 A1 (ANDRIANESIS K. [GR]) 20 July 2020
- US 2019/091040 A1 (GILL H. [GB] et al.) 28 March 2019

### Summary of the Invention

The general purpose of the present invention, therefore, is to indicate a prosthesis for an upper limb in which the functional commands for the mechanical limb can be generated by exploiting additional input data with respect to the only muscle contractions of the muscles present on the patient's stump, and the patient can receive a sensory feedback as rich as possible, with respect to the functionality of the mechanical limb: i.e., an additional sensory feedback with respect to the mere vision of the operation of the mechanical limb itself.

Other important purposes of the present invention concern the optimization of some aspects related to the manufacture of such a prosthesis.

In fact, the invention must propose an overall modular prosthetic system, in which the actual mechanical artificial limb substantially implements the pure operational and sensory functions; while the control functions by the patient, as well as the feedback functions provided to the patient, are managed by a subsystem as decoupled as possible from the artificial limb, with which, however, it interacts through appropriate interfaces.

In particular, it is useful to specify that the sensory feedback to which reference is made in the context of the present invention is based on two clearly distinguishable functions. A first pure sensory functionality, which consists only in producing general information associated with the interaction of the artificial limb with the environment, and making it available (in a computer format) on an appropriate interface, and a second physical feedback function towards the patient, which consists in stimulating sensitive parts of the patient, so that he can physically perceive these stimuli. It is clear that said stimulus signals, in order to be a significant sensory feedback, must obviously be deduced as a function of the information produced thanks to said first pure sensory functionality.

Other purposes that aim at optimizing the practical realization of the individual prostheses concern the prosthetic socket, which must be kept as simple as possible, but must also be exploited to accommodate some components that implement sensory and control functions of the prosthesis in its complex.

The purposes of the present invention can be achieved by realizing an upper limb prosthesis, comprising a mechanical limb able to perform operational functions and sensory functions, and a prosthetic socket adapted to act as a connection between said mechanical limb and the patient's stump to which said mechanical limb must be connected; said mechanical limb having a physical interface also prepared for acquiring at least one electrical signal suitable for controlling the operational functions of said mechanical limb, and for exposing at least one electrical signal capable of representing information about the state of said mechanical limb, possibly acquired by means of sensors of various types integrated into the limb itself.

Furthermore, said prosthetic socket is designed to house an electronic control module, which also includes a power supply battery suitable for powering the entire prosthesis, and said electronic control module being characterized in that:
✔ it comprises at least one inertial unit at least triaxial,
✔ it comprises at least one vibrating device,
✔ it has a physical interface designed to acquire at least one electrical signal from said mechanical limb and to present at least one electrical signal containing commands suitable for being transmitted to said mechanical limb,
✔ it comprises calculation means and memory means suitable for the execution of computer programs configured for
   - the processing of signals inputted from said mechanical limb,
   - the processing of signals acquired by means of said inertial unit,
   - generating commands to activate said vibrating device, and
   - generating commands suitable for controlling the movement and functionalities of said mechanical limb.

The main advantage of the present invention consists in the fact that, as will be better explained below, a prosthetic system made according to the teachings of the present invention satisfies all the main requirements for which it was conceived.

### Brief Description of the Drawings

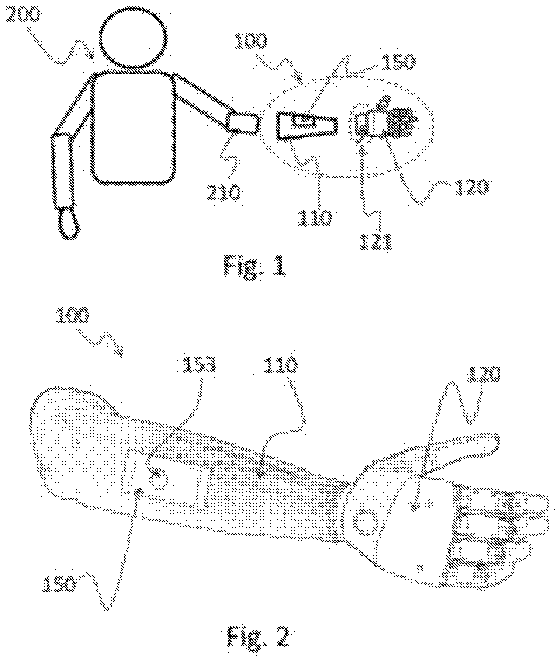

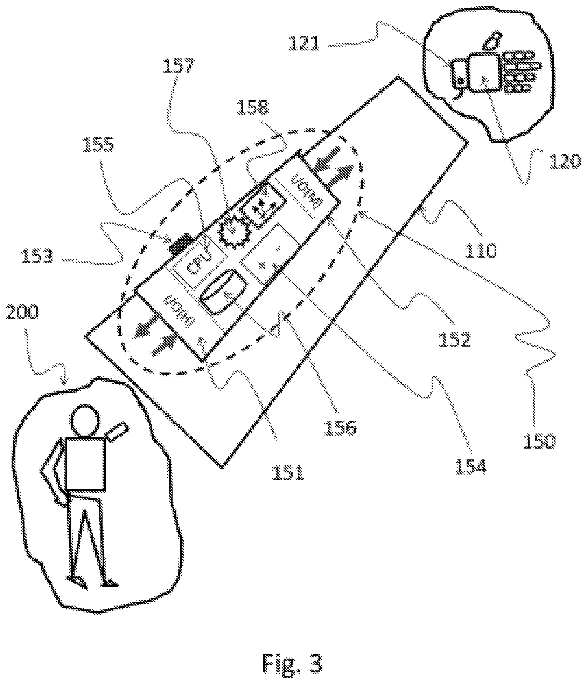

This invention has further advantages, which will become more evident from the following description, from some examples of practical embodiments which illustrate further details, from the attached claims which form an integral part of the present description, and from the attached figures in which:
✔ Figure 1 schematically shows a prosthesis of an upper limb according to the invention, in which the main subsystems that compose it are highlighted;
✔ Figure 2 shows a concrete embodiment of the present invention.
✔ Figure 3 shows, in more detail, but still schematically, the part of the prosthesis that includes the electronic control element according to the invention, in which the main subsystems that compose it are highlighted.

### Detailed Description

In Figure 1, a prosthetic system for upper limb, according to the invention, is schematically shown, it is depicted within a dashed line, and indicated as a whole with the number 100.

The number 200 indicates a generic patient who is missing a part of an upper limb; furthermore, again in Figure 1, the stump is highlighted, i.e., the terminal part of the incomplete limb to which a prosthesis must be applied; said stump is indicated in Figure 1 with the number 210.

The disability of said patient 200, exemplified in Figure 1, therefore consists in the fact that he is missing a hand as well as a part of his forearm. This type of disability represents one of the most frequent cases, and it will be taken as a reference to illustrate the present invention. However, as already mentioned, it is noted that the essential teachings of the invention can also be applied to other cases in which larger parts of an upper limb are missing. Obviously, in the event that a more extended part of the arm is missing, and the patient does not even have the elbow joint, the prosthesis will have to be differentiated in the part of the mechanical limb, which obviously must be a more complex system, but the connecting element, that is the so-called prosthetic socket, which is the element in which the essential characteristics of the invention are concentrated, can, in any case, be realized according to the teachings of the invention itself.

Said prosthetic system 100 is represented in Figure 1 by showing the subsystems that compose it detached from each other for clarity of illustration. The number 110 indicates the prosthetic socket, while the number 120 indicates the mechanical limb, which, in the case considered, is a generic artificial hand.

The known art, of course, offers both many artificial hands and prosthetic sockets. The prosthetic system 100 according to the invention can integrate an artificial limb (for example, an artificial hand) made according to the known technique.

With reference to the known technique, it is observed that a number artificial limbs are available. In particular there are very sophisticated artificial hands, with very high performance, both from the point of view of the actions they are able to perform and from the point of view of sensory feedback.

A product that can be considered representative of the state of the art, as far as artificial hands are concerned, is the hand proposed by the Scottish "Touch Bionics". It is a hand with five fingers, morphologically very similar to a natural hand, which can perform various manipulations and types of grip, similar to those that can be performed by natural hands. One of the main problems that limits the use of very complex poly-articulated artificial hands lies in the fact that the most sophisticated systems suffer from the limitations given by the possibilities of interaction between the artificial limb and the patient. It could be summarized that it is useless to have a hyper-sensorized mechanical limb that can perfectly replicate the abilities of a natural hand, but which cannot be effectively controlled by the patient; and this problem, as also anticipated in the first part of this description, is still largely unsolved.

In the case of the artificial hand proposed by "Touch Bionics", there are integrated various devices for the control: this hand therefore interprets various types of commands, which can be given by the patient. Particularly innovative, at the time of their introduction, were commands obtained through an accelerometer positioned in the central part of the artificial hand. Thanks to this accelerometer, the patient can move his hand according to some coded movements, (and which must therefore be learned by the patient) which are interpreted as commands: for example, to produce the opening or closing of the hand, or to control individual fingers.

In any case, it is not the subject of this description to further investigate the techniques at the state of the art, except to observe how the known art proposes systems that integrate most of the operating technologies into the artificial limb, including those for the control of the movements of the limb itself.

Similar considerations can be made with reference to sensory functions. Typical sensory feedback are sounds and vibrations, the latter are perceived by the patient through the propagation that crosses the prosthetic socket, with all the problems already mentioned due to the fact that the vibration is attenuated in propagation (remember that, for reasons of comfort, often the prosthetic socket is coupled to the stump with fairly soft parts) reducing the precision of the information that is conveyed by a feedback based on a vibration generated in the prosthetic limb.

Having made these preliminary considerations on the mechanical limbs proposed by the known art, it is highlighted a first characteristic of the mechanical limb 120, suitable to be used in the invention. Although known and even different mechanical limbs can be used, the mechanical limb 120 suitable to be used in the invention does not require components for detecting control commands (such as accelerometers), nor components for generating sensory feedback (such as, for example, vibrating elements or sound buzzers). What the mechanical limb 120 needs, in order to implement the invention, is a physical communication interface.

Said physical communication interface, is indicated in Figure 1 with the number 121, and consists of a set of physical contacts, i.e., a communication port, which can be connected to suitable wiring. The mechanical limb 120 is configured to receive commands, through said physical communication interface 121, in order to perform all the movements for which it is designed. In addition, this interface uses signals, encoded through a predefined protocol and language, which describe the state of the artificial limb in a substantially exhaustive manner.

In summary, the artificial limb 120 presented in Figure 1 is characterized, among other things, by the fact that it has an interface 121 on which some coded information regarding the limb itself is represented. Some examples of this information are the position assumed by the limb, as well as the forces it is exerting on what it is touching (force of the grips, intensity of the thrusts, etc.), but also any other information acquired by other sensors possibly integrated in the limb itself.

This implementation choice makes it possible to very clearly decouple the sensory and operational functions of the limb 120 from their control, so as to couple the artificial limb to different types of patients 200, with different control attitudes.

In theory, some control commands could also be generated automatically, and not directly from a patient action 200. For example, if the patient is engaged in a manipulation of fragile objects, he could set an operating mode (for example through a voice command) that prevents the limb from exercising too vigorous grips: in in this case, the control of the limb would take place with corrections with respect to the commands given with direct patient actions, to prevent grips that could damage the manipulated object.

The choice of using a mechanical limb 120 with a rich interface 121, and which supports communications in both directions: input commands and information for sensory feedback at the output, on the one hand simplifies the mechanical limb 120 which is only required to implement operational and sensory functions, but does not need to implement interpretative functions or to generate signals in such a form as to offer physical feedback to the patient, like emissions of sounds or generation of vibrations.

On the other hand, this simplification of the mechanical limb 120 requires the presence of an additional electronic control module. According to the teachings of the invention, said electronic control module is housed in an appropriate zone of the prosthetic socket 110, and it is indicated in Figure 1 with the number 150.

It is observed that the electronic processor modules are now very miniaturized and low-consuming, just as the batteries are also very miniaturized; so that it can be stated that the choice of the positioning of said control module 150, although being a choice to be made on a case-by-case basis, given that the prosthetic sockets are, and remain, substantially ad-hoc-made objects, it isn't, for sure, a choice heralding problems: a convenient position for placing said control module 150, in the prosthetic socket 110, is easy to identify in all practical cases.

Said control module 150 represents the most distinctive feature of the present invention. It contains one or more batteries, but also performs a set of other functions that allow optimal control of the mechanical limb 120.

Figure 2 shows a real embodiment of the present invention. In Figure 2 the three main modules of the upper limb prosthetic system 100 according to the invention are visible, and are indicated with the same numbers used in Figure 1, they are the actual mechanical limb 120 (in this case an artificial hand), the prosthetic socket 110, and the control module 150.

The first feature that can be seen in the implementation form presented in Figure 2 is the possibility of making the prosthetic system 100 in realistic shape and proportions.

To be noted that the three main modules (mechanical limb 120, prosthetic socket 110 and control module 150) are, from the point of view of their couplings, clearly distinguishable and separable even by means of simple release and hooking mechanisms. This last feature is a direct consequence of the modular design of the prosthetic system 100.

Finally, it is also noted how the customized part of the prosthetic system 100 is substantially confined to the prosthetic socket. Effectively, the artificial hand 120 can take on various sizes, however, the variety of hand size can be absolutely managed by proposing a very small number of predefined sizes (for example 3 different sizes), and therefore industrially manufactured and not made to measure: consequently, with these, it is possible to satisfy almost all needs. In fact, the aesthetic aspect linked to the proportions between the two arms, and between the patient's arm and body, can be managed by acting only on the conformation of the prosthetic socket 110.

In the control element 150 there is also highlighted, indicated with the number 153, a control button that can be operated by the patient 200 with the other hand. The opportunity to make a further manual control interface available is, once again, attributable to the modular and decoupled design of the entire prosthetic system 100, and to the fact that said control element 150, due to its dimensions and conformation, can be easily placed in the prosthetic socket 110.

Figure 3 shows, in more detail, said control module 150, highlighting the main characteristics that make the present invention truly original. In fact, the modular design of the prosthetic system 100 is not just a physical modularity of the couplings, but also, and above all, a functional modularity.

Figure 3 is again a schematic representation in which the proportions are evidently lost (after all, a realistic representation has been provided with absolute evidence in Figure 2). The mechanical limb 120 and the patient 200, without a part of one of his upper limbs, are represented in a symbolic way as Figure 3 aims to focus attention essentially on the control module, in turn embedded in the prosthetic socket 110

It should be noted that also in Figure 3, where possible, the same numbering used in the previous Figures 1 and 2 has been maintained to indicate the various elements of the prosthetic system 100: only the control module, which, as a whole, is always indicated with the number 150 (being highlighted also within an area delimited by a dashed line), in Figure 3, is presented in greater detail, highlighting its main components, all indicated with numbers belonging to the 15x decade.

Before listing the essential parts that make up said control element 150, it is specified, as has already said, that the mechanical limb 120 (in the exemplified case, an artificial hand), in theory, can be an interchangeable module, provided that it offers an open interface 121 towards said control element 150. Therefore, not only the patient 200 could benefit from different limbs 120 depending on the activities he has to perform, for example using a working limb and a leisure limb, but he could also use limbs from different manufacturers, as long as these provide an open interface 121. Being said open interface 121 characterized both from the physical point of view (connectors, signal levels, etc.) and from the point of view of the communication protocols; that is, the commands necessary for its functional control must be specified, as well as the format of the status information that is available on the interface itself.

Again, with reference to said open interface 121, it is observed that, being bidirectional, it lends itself to a functional control in a closed chain that can be carried out through said control element 150; unlike the typical cases in the prior art, in which the control from the outside of the mechanical limb 120 always takes place in an open chain, wherein any eventual control in closed chain is always implemented with controllers specifically integrated into the mechanical limb 120 itself.

The numbers 151 and 152 represent the interfaces of said control element 150. In particular, the number 151 indicates the interface between said control element 150 and the patient 200, while the number 151 indicates the interface between said control element 150 and the mechanical limb 102. Both interfaces 151 and 152 are bidirectional and programmable. In a preferred implementation form, in addition to being programmable the protocol with which said interfaces communicate with the respective entities to which they are interfaced, they can also be set with respect to some physical parameters, possibly being able to adjust the physical levels of the signals used for communication, so as to be usable in coupling with different physical interfaces possibly present on the devices to which they are coupled. This performance can be useful both from the side of the mechanical limb 120 (interface I/O(M) 152), and from the side of the patient 200 (interface I/O(H) 151); the latter possibility, in particular, can be interesting to add any devices to enrich the set of sensory feedback returned to the patient 200, and/or to acquire additional commands from the patient himself, being able to resort to devices possibly proposed by different suppliers.

It should be noted that an actual implementation of the two I/O(M) 152 and I/O(H) 151 interfaces can be implemented according to multiple variants, all constituting different forms of implementation of the same invention. It is also noted that the distinction between the two I/O(M) 152 and I/O(H) 151 interfaces, respectively towards the mechanical limb 120 or towards the patient 200, is a functional distinction that does not necessarily correspond to a physical distinction, given that these interfaces can be physically implemented by means of a "bus" architecture, in which the entities to which they are connected depend on the information contained in the exchanged signal and not on the physical connection.

The functional commands to be transmitted to the mechanical limb 120 must be interpreted by the control element 150, but must obviously be the effect of the will of the patient 200, and must therefore be generated as a consequence of input information somehow produced by the patient. Therefore, from the physical point of view, said interface and I/O(H) 151 is designed to acquire at least one electrical signal from any sensors designed to detect voluntary physical activities by the patient 200.

As regards the control commands, which must originate from a decision of the patient 200, they can be synthesized both starting from signals acquired (as previously mentioned) through the interface 151 [I/O(H)], and both from data generated internally by the control module 150 itself. In fact, in a preferred form of implementation, said control element 150 also includes at least one triaxial inertial unit, indicated in Figure 3 with the number 158, which detects the movements of the forearm. In this way, the patient 200 is able to use a sort of command language by carrying out some pre-coded movements (and typically calibrated on the individual patient) which are recognized by the control unit 150, interpreted, and synthesized into functional commands to be transmitted to the mechanical limb 120 through the interface 152 [I/O(M)], for this purpose.

The presence of said inertial unit 158 is also useful for supporting calibration functions of the myoelectric commands which must be interpreted correctly also taking into account the position of the patient's arm; in this regard, it is cited by the same authors of this patent application, IT 102020000023011, ["Prosthetic device with myoelectric control and method for the calibration and use of said device"].

Sensory feedback can also be transmitted to the patient 200 both by means of appropriate sensory stimulation devices that can be activated via the interface 151 [I/O(H)], and by means of sensory stimuli internally generated in the control unit 150.

In particular, said interface 151 [I/O(H)] is designed to present at least one electrical signal containing commands suitable for activating suitable sensory stimulation devices applied to sensitive parts of the body of said patient 200.

Again, with reference to the modalities for returning sensory feedback, in a preferred embodiment of the invention, said control element 150 also comprises a vibrating element, indicated in Figure 3 with the number 157, which generates precisely a vibration which propagates through the structure of the prosthetic socket 110 and can be sensed by the patient's stump 200.

The possibility of acquiring signals coming from the patient's body 200, and of sending signals towards the patient's body 200, in an absolutely general way, is one of the most advantageous features of the present invention. In fact, both the sensory capacity and the muscular activity present on the stumps of patients who have lost a limb, or who are without one since birth, are individual characteristics of each patient, and should, in general, be treated case by case, as different characteristics.

Since one of the fundamental requirements of the invention is precisely that of decoupling all standardizable performances from those requiring customizations, the possibility of having a control element 150 equipped with an open interface 151 [I/O(H)], allows to satisfy this need, possibly being able to conceive separately devices suitable for returning personalized sensory feedback or devices suitable for acquiring command stimuli which are also personalized.

For example, in the case of a patient 200 whose stump is completely numb, and has no controllable musculature, so that the patient is unable to exercise suitable muscle contractions to formulate commands for the mechanical limb through contractions of muscles in contact with the prosthetic socket 110, both the command signals and the sensory feedback could be reported to other parts of the patient's body, through sensors and stimulators worn, or applied, in other parts of the patient's body and, in turn, connected to the control unit 150, through said open interface 151 [I/O(H)].

After all, the fact of using efficient body parts to control artificial limbs is not a completely new idea; in fact, before the advent of electronics, (and also in many prostheses still used today) a minimum of functional control of the mechanical limbs can be exercised through belts, tie rods and mechanisms based on levers, pulleys and various gears designed to bring back the movements of the mechanical limb to movements of other parts of the body with greater functionality.

From the just proposed description of the control element 150, it is clear that this control element 150 is a programmable microprocessor device. In fact, it processes data, generates commands and controls input and output information. In Figure 3 there are also highlighted calculation means, indicated with the number 155, and memory means, indicated, with the number 156 (i.e., a microprocessor board, typically made with integrated circuits that reach very high levels of miniaturization).

Said control element 150, in fact, is therefore also a programmable electronic computer equipped with a battery, indicated in Figure 3 with the number 156. It can be observed that said battery 156 is necessary, in addition to powering the control element 150 itself, also to power the mechanical limb 120 and all the devices that need to be powered in the prosthetic system 100 according to the invention (for example sensors and sensory devices). Among other things, the preferred location of said control element 150, i.e., embedded in the prosthetic socket 110, is the typical location of the batteries that are obviously necessary for any modern mechanical prosthesis, even those according to the known technique. The fact that this location can also be maintained for a much more complex system than a simple battery, such as the control element 150 according to the invention, is a consequence of the considerable progress made with respect to the miniaturization of electronics, as well as batteries.

With reference to the computational part of said control element 150, the most important thing to underline is that, it too, as in the philosophy with which the whole system was conceived, is an element designed to support the installation of programs and their execution.

Therefore, each device connected to said control element 150 through its interfaces 151 and 152, either a specific mechanical limb 120, or a device that supports interaction with the patient 200, can be managed by installing and executing a suitable program. Evidently, said control element 150 must therefore be equipped also with a control interface suitable for loading programs, maintenance and diagnostics of the device as a whole. We do not dwell on the innumerable ways in which said control interface can be implemented, since the known art manages this type of requirement with absolute efficiency, for the maintenance of each microprocessor device.

It is only observed how a wireless interface, preferably with a short-range communication (for example NFC: Near Field Communication or "Bluetooth" in its various versions), appears particularly convenient, in particular to support diagnostic applications on the system status, as a whole, or limited to parts of it.

For example, through the analysis of the data used for the closed-chain control of mechanical actuators, it is possible to deduce the possible state of wear of certain mechanisms, the possible presence of friction with values other than the optimal ones or, trivially, the state of charge of battery 156, etc...

Depending on the different forms of implementation, the main diagnostic outcomes can be signaled by means of luminous or sound indicators, or small monitors, which can also be integrated into said control module 150.

Finally, still with regard to control interfaces, Figure 3 (as well as Figure 2) highlights the presence of a multifunction button, indicated with the number 153, which can be operated manually to turn off the control element 150, or to perform a restart, or for the quick implementation of other potentially useful actions.

### Variants and Concluding Remarks

The upper limb prosthesis 100 according to the teachings of the invention allows to maintain some methods of making and applying a prosthesis typical of the prior art.

In other words, is not substantially upset the process which involves choosing an artificial hand or limb 120 available from a vast set of known mechanical limbs, and adapting it to the needs of a patient 200 by means of a prosthetic socket 110 of simple manufacture, but made to measure.

However, the invention makes it possible to better exploit the functions of the artificial limb 120, delegating its control to a processor module 150 equipped with appropriate interfaces. Said control module 150, as also occurs in various known embodiments, is arranged to receive commands generated by the patient 200 through contractions of the muscles which are active in correspondence with the stump 210, and which are detectable by means of suitable sensors positioned in the area in which the prosthetic socket 110 is coupled to the stump 210 of the patient 200.

However, said control module 150 is able to manage also other forms of input, thanks to the fact that it integrates other components that detect voluntary actions of the patient 200, such as for example small displacements of the forearm, detected by means of one or more inertial units (for example: gyroscopes, magnetometers, or accelerometers made with various technologies, etc.).

But above all, said control module 150 is equipped with an open and programmable interface that can acquire multiple other signals generated by the patient with the intent of controlling his prosthesis.

In different embodiments, said control module 150 can also receive and process other forms of interaction with the patient not yet mentioned in the present description.

For example, said control module 150 may also include a microphone device so as to be able to acquire voice commands from the patient 200, through the execution of programs (now mature, and at the state of the art) of voice recognition.

In general, said control element 150 is a full-fledged computing unit, which may eventually support an open operating system, i.e., suitable for the installation and execution of always new programs, able to manage other further forms of interaction, at the moment not yet identified, between patient 200 and mechanical limb 120.

It is noted that the architecture of this computing unit can also be implemented according to many architectural models, being also possible distributed models in which the computing means are composed of a plurality of electronic processors. The management interfaces of the aforementioned computing unit can also be implemented according to different variants: in a preferred embodiment, said control module 150 can be configured and maintained through a wireless connection (for example Bluetooth) with an external computer; or, in simpler forms of implementation, a port (for example USB) can be provided to ensure a cable connection.

In any case, both the different computing architectures and the access methods to configure, maintain and monitor the computing unit used by said control module 150, must all be considered different forms of implementation of the same invention.

Among the experimental technologies that aim at implementing new (and possibly more effective) forms of interaction between the patient 200 and said control module 150, some solutions that interface electronic devices with nervous system terminations seem interesting: these technologies, although not yet mature, look promising, and could be exploited to generate mechanical limb controls with accuracy and complexity unthinkable today.

The prosthetic system 100 according to the invention, thanks to its conceptual modularity, is already designed to integrate any type of technology that develops advanced interactions with a patient's body.

The invention was conceived explicitly thinking of prostheses for upper limbs, since, in this type of prosthesis, both problems related to functional control and to sensoriality arise, in a particularly accentuated way; and to the perception that the patient has in general of his artificial limb. In fact, the natural hand, from a purely mechanical point of view, is an extremely complex system rich in possible movements, but it is also a part of the body that hosts many sensory functions: after all, manipulations and touch represent one of the main modalities with which men come into contact with the external physical world.

However, the validity of the teachings of the present invention is not structurally confined to the field of upper limb prostheses. In fact, the invention defines a prosthetic platform designed so that new performances (both in functional and sensory fields) can be implemented in an almost continuous way, and for this reason it is very interesting in the context of upper limb prostheses, however, it is observed that the main teachings of the invention can be generally applied in all prostheses in which a mechanical part equipped with a suitable interface (such as the interface 121 described in the present invention) is connected to the patient's body through a prosthetic socket suitable for housing a control unit, such as the control module 150 described in the present invention.

What characterizes the invention, in fact, is the system of interfaces between sensors which detect voluntary acts of the patient 200 and said control unit 150, and between said control unit 150 and the controlled mechanical limb 120. This intermediary function, besides to allow the identification, depending on the case, of the best solution for each individual patient, it also works in the opposite direction, that is, transmitting sensory feedback from the artificial limb 120 to the patient.

Also in this case, the sensory feedback information is first acquired by said control module 150, in the form of computer data, and then is returned to the patient's perception, according to the feedback devices provided. A vibrating element is certainly integrated in the control module 150, being the vibratory feedback a very effective form of feedback and used in the prosthetic technique. The advantage of having the vibratory element positioned in the prosthetic socket 110, in a position closer to the stump 210, allows the patient 200 to perceive with greater precision any modulations in the intensity of the vibration.

In some variants, embodiments may be proposed in which the section of the socket 110 between the vibrating element and the stump is stiffened to improve the propagation of the vibration, or the vibrating elements can even be physically positioned in contact with the stump 210, and activated by means of the control interfaces with which the control module 150 is equipped, and which make it suitable for communicating in both directions, to and from the patient 200, and to and from the artificial limb. In this way, the prosthetic system 100 as a whole is allowed to be configured with a versatility not yet present in prior art solutions.

Other variants of the present invention relate to the possibility of returning to the patient 200, for example by means of suitable wearable devices, also other forms of sensory feedback, possibly involving more sensitive parts of the patient's body. For example, feedback based on temperature, or small electrical stimulations can be used or, why not, once again using technologies capable of interacting directly with the nervous system.

In an evolutionary context, in which artificial intelligence applications will have a growing impact, the prosthetic system 100 according to the invention appears to be already prepared to benefit from any technological progress in this field. In fact, both the operational commands to be given to the mechanical limb 120, and the sensory feedbacks are generated as a result of appropriate programs performed at the level of the control module 150.

Therefore, if the available information is enriched as a result of new technological opportunities, or if automatic learning programs are able to synthesize, or predict, more precisely the patient's intentions, the operation of the prosthesis could benefit from it while maintaining the architecture and the essential features taught in the present invention.

## Claims

1. An upper limb modular prosthetic system (100), comprising
- a mechanical artificial limb (120) able to perform operational functions and sensory functions, and
- a prosthetic socket (110) adapted to act as a connection between said mechanical artificial limb (120) and the stump (210) of the patient (200) to which said mechanical limb (120) must be connected;
said mechanical artificial limb (120) having a physical interface (121) also prepared for
- acquiring at least one electrical signal suitable for controlling the operational functions of said mechanical artificial limb (120), and
- exposing at least one electrical signal capable of representing information about the state of said mechanical limb (120);
and said prosthetic socket (110) being designed to house an electronic control module (150), also comprised in said upper limb modular prosthetic system (100);
and said electronic control module (150) is **characterized in that** it physically integrates together at least:
a. a power supply battery (154) suitable for powering the whole of said prosthetic system (100),
b. one inertial unit at least triaxial (158),
c. one vibrating device (157),
d. a physical interface (152) designed to acquire at least one electrical signal from said mechanical limb (120) and to present at least one electrical signal containing commands suitable for being transmitted to said mechanical limb (120),
e. calculation means (155) and memory means (156) suitable for the execution of computer programs configured for
i. the processing of signals inputted from said mechanical limb (120),
ii. the processing of signals acquired by means of said inertial unit (158),
iii. generating commands to activate said vibrating device (157) and
iv. generating commands suitable for controlling the movement and functionalities of said mechanical limb (120).

2. Modular prosthetic system (100) according to claim 1 wherein said control module (150) is also **characterized in that**:
✔ it has a physical interface (151) designed to acquire at least one electrical signal inputted from at least one sensor designed to detect voluntary physical activities by said patient (200), and
✔ said calculation means (155) and memory means (156) are designed to execute computer programs configured to generate commands suitable for controlling the movement and the functionalities of said mechanical limb (120) also as a function of said at least one electrical signal inputted from said sensors.

3. Modular prosthetic system (100) according to claim 1 wherein said control module (150) is also **characterized in that**:
it has a physical interface (151) designed to present at least one electrical signal containing commands suitable for activating one or more sensorial stimulation devices applied to sensitive parts of the body of said patient (200), and
✔ said calculation means (155) and memory means (156) are designed to execute computer programs configured to generate said commands suitable for activating said one or more sensorial stimulation devices as a function of said at least one electrical signal inputted from said mechanical limb (120).

4. Modular prosthetic system (100) according to claim 2 or claim 3 in which said control module (150) is also **characterized in that** said calculation means (155) and memory means (156) are suitable for supporting an operating system, in turn suitable for the installation of computer programs configured to program said physical interfaces (151) e (152).

5. Modular prosthetic system (100) according to claim 1 wherein said control module (150) is also **characterized in that** it comprises a multifunction button (153) that can be operated manually by the patient (200).

6. Modular prosthetic system (100) according to claim 1 wherein said control module (150) is also configured to carry out diagnostic programs about the state of said mechanical limb (120), as a function of said at least one electrical signal suitable for representing information about the state of said mechanical limb (120).

7. Modular prosthetic system (100) according to claim 1 in which said control module (150) is also **characterized in that** it comprises a microphone device designed to acquire voice commands from the patient (200).

## Patentansprüche

1. Ein modulares Prothesensystem (100) für die oberen Gliedmaßen, bestehend aus
- eine mechanische künstliche Gliedmaße (120), die operative Funktionen und sensorische Funktionen erfüllen kann, und
- eine Prothesenfassung (110), die als Verbindung zwischen der mechanischen künstlichen Gliedmaße (120) und dem Stumpf (210) des Patienten (200) dient, an den die mechanische Gliedmaße (120) angeschlossen werden muss;
wobei die mechanische künstliche Gliedmaße (120) eine physikalische Schnittstelle (121) aufweist, die ebenfalls für
- Erfassen mindestens eines elektrischen Signals, das zur Steuerung der Betriebsfunktionen des mechanischen künstlichen Glieds (120) geeignet ist, und
- Ausgeben mindestens eines elektrischen Signals, das Informationen über den Zustand der mechanischen Gliedmaße (120) darstellen kann;
und der Prothesenschaft (110) ist so gestaltet, dass er ein elektronisches Steuermodul (150) aufnimmt, das ebenfalls in dem modularen Prothesensystem (100) für die oberen Gliedmaßen enthalten ist;
und das elektronische Steuermodul (150) ist **dadurch gekennzeichnet, dass** es physikalisch mindestens:
a. eine Stromversorgungsbatterie (154), die für die Stromversorgung des gesamten Prothesensystems (100) geeignet ist,
b. eine mindestens dreiachsige Trägheitseinheit (158),
c. ein Vibrationsgerät (157),
d. eine physikalische Schnittstelle (152), die dazu ausgelegt ist, mindestens ein elektrisches Signal von dem mechanischen Gliedmaße (120) zu erfassen und mindestens ein elektrisches Signal bereitzustellen, das Befehle enthält, die zur Übertragung an das mechanische Gliedmaße (120) geeignet sind,
e. Berechnungsmittel (155) und Speichermittel (156), die für die Ausführung von Computerprogrammen geeignet sind, die für
i. die Verarbeitung der von der mechanischen Gliedmaße (120) eingegebenen Signale,
ii. die Verarbeitung der mittels der Trägheitseinheit (158) erfassten Signale,
iii. Erzeugen von Befehlen zum Aktivieren der Vibrationsvorrichtung (157) und
iv. Generieren von Befehlen, die zum Steuern der Bewegung und der Funktionen des mechanischen Glieds (120) geeignet sind.

2. Modulares Prothesensystem (100) nach Anspruch 1, wobei das Steuermodul (150) außerdem **dadurch gekennzeichnet ist, dass**:
✔ es verfügt über eine physikalische Schnittstelle (151), die dazu ausgelegt ist, mindestens ein elektrisches Signal zu erfassen, das von mindestens einem Sensor eingegeben wird, der dazu ausgelegt ist, freiwillige körperliche Aktivitäten des Patienten (200) zu erfassen, und
✔ die Berechnungsmittel (155) und Speichermittel (156) sind dazu ausgelegt, Computerprogramme auszuführen, die dazu konfiguriert sind, Befehle zu erzeugen, die zur Steuerung der Bewegung und der Funktionalitäten der mechanischen Gliedmaße (120) geeignet sind, auch als Funktion des mindestens einen elektrischen Signals, das von den Sensoren eingegeben wird.

3. Modulares Prothesensystem (100) nach Anspruch 1, wobei das Steuermodul (150) außerdem **dadurch gekennzeichnet ist, dass**:
✔ es verfügt über eine physikalische Schnittstelle (151), die dazu ausgelegt ist, mindestens ein elektrisches Signal mit Befehlen bereitzustellen, die zur Aktivierung eines oder mehrerer sensorischer Stimulationsgeräte geeignet sind, die an empfindlichen Körperteilen des Patienten (200) angebracht sind, und
✔ die Berechnungsmittel (155) und Speichermittel (156) sind dazu ausgelegt, Computerprogramme auszuführen, die dazu konfiguriert sind, die Befehle zu erzeugen, die zum Aktivieren des einen oder der mehreren sensorischen Stimulationsgeräte als Funktion des mindestens einen elektrischen Signals geeignet sind, das von der mechanischen Gliedmaße (120) eingegeben wird..

4. Modulares Prothesensystem (100) nach Anspruch 2 oder 3, wobei das Steuermodul (150) außerdem **dadurch gekennzeichnet ist, dass** die Rechenmittel (155) und Speichermittel (156) zur Unterstützung eines Betriebssystems geeignet sind, das wiederum zur Installation von Computerprogrammen geeignet ist, die zur Programmierung der physikalischen Schnittstellen (151) und (152) konfiguriert sind.

5. Modulares Prothesensystem (100) nach Anspruch 1, wobei das Steuermodul (150) außerdem **dadurch gekennzeichnet ist, dass** es einen Multifunktionsknopf (153) umfasst, der vom Patienten (200) manuell bedient werden kann.

6. Modulares Prothesensystem (100) nach Anspruch 1, wobei das Steuermodul (150) außerdem so konfiguriert ist, dass es Diagnoseprogramme über den Zustand des mechanischen Glieds (120) als Funktion des mindestens einen elektrischen Signals ausführt, das geeignet ist, Informationen über den Zustand des mechanischen Glieds (120) darzustellen.

7. Modulares Prothesensystem (100) nach Anspruch 1, wobei das Steuermodul (150) außerdem **dadurch gekennzeichnet ist, dass** es eine Mikrofonvorrichtung umfasst, die dazu ausgelegt ist, Sprachbefehle vom Patienten (200) zu erfassen.

## Revendications

1. Un système prothétique modulaire de membre supérieur (100), comprenant
- un membre artificiel mécanique (120) capable d'exécuter des fonctions opérationnelles et des fonctions sensorielles, et
- une emboîture prothétique (110) adaptée pour servir de connexion entre ledit membre artificiel mécanique (120) et le moignon (210) du patient (200) auquel ledit membre mécanique (120) doit être connecté;
ledit membre artificiel mécanique (120) ayant une interface physique (121) également préparée pour
- acquérir au moins un signal électrique adapté pour contrôler les fonctions opérationnelles dudit membre artificiel mécanique (120), et
- exposer au moins un signal électrique capable de représenter des informations sur l'état dudit membre mécanique (120);
et ladite emboîture prothétique (110) étant conçue pour loger un module de commande électronique (150), également compris dans ledit système prothétique modulaire de membre supérieur (100);
et ledit module de commande électronique (150) est **caractérisé en ce qu'**il intègre physiquement ensemble au moins:
a. une batterie d'alimentation (154) adaptée pour alimenter l'ensemble dudit système prothétique (100),
b. une centrale inertielle au moins triaxiale (158),
c. un appareil vibrant (157),
d. une interface physique (152) conçue pour acquérir au moins un signal électrique provenant dudit membre mécanique (120) et pour présenter au moins un signal électrique contenant des commandes aptes à être transmises audit membre mécanique (120),
e. des moyens de calcul (155) et des moyens de mémoire (156) adaptés à l'exécution de programmes informatiques configurés pour
i. le traitement des signaux provenant dudit membre mécanique (120),
ii. le traitement des signaux acquis au moyen de ladite centrale inertielle (158),
iii. générer des commandes pour activer ledit dispositif vibrant (157) et
iv. générer des commandes adaptées pour contrôler le mouvement et les fonctionnalités dudit membre mécanique (120).

2. Système prothétique modulaire (100) selon la revendication 1, dans lequel ledit module de commande (150) est également **caractérisé en ce que**:
✔ il comporte une interface physique (151) conçue pour acquérir au moins un signal électrique provenant d'au moins un capteur conçu pour détecter les activités physiques volontaires dudit patient (200), et
✔ lesdits moyens de calcul (155) et moyens de mémoire (156) sont conçus pour exécuter des programmes informatiques configurés pour générer des commandes adaptées au contrôle du mouvement et des fonctionnalités dudit membre mécanique (120) également en fonction dudit au moins un signal électrique provenant desdits capteurs.

3. Système prothétique modulaire (100) selon la revendication 1, dans lequel ledit module de commande (150) est également **caractérisé en ce que**:
✔ il dispose d'une interface physique (151) conçue pour présenter au moins un signal électrique contenant des commandes aptes à activer un ou plusieurs dispositifs de stimulation sensorielle appliqués à des parties sensibles du corps dudit patient (200), et
✔ lesdits moyens de calcul (155) et moyens de mémoire (156) sont conçus pour exécuter des programmes informatiques configurés pour générer lesdites commandes adaptées à l'activation dudit ou desdits dispositifs de stimulation sensorielle en fonction dudit au moins un signal électrique entré depuis ledit membre mécanique (120).

4. Système prothétique modulaire (100) selon la revendication 2 ou la revendication 3, dans lequel ledit module de commande (150) est également **caractérisé en ce que** lesdits moyens de calcul (155) et moyens de mémoire (156) sont adaptés pour supporter un système d'exploitation, à son tour adapté à l'installation de programmes informatiques configurés pour programmer lesdites interfaces physiques (151) et (152)..

5. Système prothétique modulaire (100) selon la revendication 1, dans lequel ledit module de commande (150) est également **caractérisé en ce qu'**il comprend un bouton multifonction (153) pouvant être actionné manuellement par le patient (200).

6. Système prothétique modulaire (100) selon la revendication 1, dans lequel ledit module de commande (150) est également configuré pour exécuter des programmes de diagnostic sur l'état dudit membre mécanique (120), en fonction dudit au moins un signal électrique apte à représenter des informations sur l'état dudit membre mécanique (120)..

7. Système prothétique modulaire (100) selon la revendication 1, dans lequel ledit module de commande (150) est également **caractérisé en ce qu'**il comprend un dispositif de microphone conçu pour acquérir des commandes vocales du patient (200)..
